**Europäisches Patentamt**

**European Patent Office**

**Office européen des brevets**

(11) Publication number: **0 160 335**
**B1**

# EUROPEAN PATENT SPECIFICATION

(45) Date of publication of patent specification: **25.01.89**

(21) Application number: **85200587.5**

(22) Date of filing: **17.04.85**

(51) Int. Cl.⁴: **C 07 C 2/00, B 01 J 29/04**

(54) Process for the preparation of an aromatic hydrocarbon mixture.

(30) Priority: **02.05.84 NL 8401384**

(43) Date of publication of application:
**06.11.85 Bulletin 85/45**

(45) Publication of the grant of the patent:
**25.01.89 Bulletin 89/04**

(84) Designated Contracting States:
**BE CH DE FR GB IT LI NL SE**

(56) References cited:
**EP-A-0 021 475**
**EP-A-0 107 875**
**EP-A-0 107 876**
**DE-A-2 755 770**
**FR-A-2 374 283**
**US-A-4 002 578**

(73) Proprietor: **SHELL INTERNATIONALE
RESEARCH MAATSCHAPPIJ B.V.
Carel van Bylandtlaan 30
NL-2596 HR Den Haag (NL)**

㉻ The inventor has agreed to waive his entitlement
to designation

(74) Representative: **Aalbers, Onno et al
P.O. Box 302
NL-2501 CH The Hague (NL)**

Courier Press, Leamington Spa, England.

# EP 0 160 335 B1

**Description**

The invention relates to a process for the preparation of an aromatic hydrocarbon mixture from mono-olefins with two to four carbon atoms per molecule or from aliphatic hydrocarbon mixtures, at least 50 wt.% of which consists of the said olefins.

Mono-olefins with two to four carbon atoms per molecule can be converted at a temperature of 325—700°C into aromatic hydrocarbon mixtures by contacting the olefins with a catalyst containing a crystalline aluminium silicate with a special structure. Such crystalline aluminium silicates are characterized by the fact that after calcination in air for one hour at 500°c they possess the following distinguishing features:

a) an X-ray powder diffraction pattern containing as strongest lines the four lines listed in Table A,

<u>TABLE A</u>

| $d(\text{Å})$ |
| --- |
| $11.1 \pm 0.1$ |
| $10.0 \pm 0.1$ |
| $3.84 \pm 0.07$ |
| $3.72 \pm 0.06$ |

and,

b) in the formula, giving the composition of the silicate expressed in moles of the oxides, the $SiO_2/Al_2O_3$ mol ratio is 25—400.

The above-mentioned process results in a product in which, besides the desired $C_5^+$ hydrocarbons, $C_4^-$ hydrocarbons formed as byproducts also occur. The $C_5^+$ fraction present in the product is more valuable according as it has a higher aromatic content.

Experimental research into the above-mentioned process has shown that the aromatic content of the $C_5^+$ fraction obtained depends to a large extent on the chosen reaction temperature and $SiO_2/Al_2O_3$ mol ratio of the crystalline silicate. According as a higher reaction temperature and/or a crystalline silicate with a lower $SiO_2/Al_2O_3$ mol ratio is used in the process, a product is obtained whose $C_5^+$ fraction has a higher aromatic content. For both these measures, however, the preparation of a product whose $C_5^+$ fraction has a higher aromatic content is accompanied by a reduction in the yield of the $C_5^+$ fraction. The research showed that a particular relationship exists between the increase in the aromatic content of the $C_5^+$ fraction and the reduction in the yield of the $C_5^+$ fraction, so that, in principle, for every aromatic content of the $C_5^+$ fraction being prepared, there is a particular potential yield of that $C_5^+$ fraction. This relationship is unfavourable to the extent that only low yields of $C_5^+$ fractions with a relatively high aromatic content can be obtained. Since a possible application of the process on a commercial scale depends not only on the aromatic content of the $C_5^+$ fraction, but also on the potential yield of that $C_5^+$ fraction, there is a practical limit to the maximum aromatic content of the $C_5^+$ fraction being prepared, due to the fact that the corresponding yield of the $C_5^+$ fraction must still be acceptable.

Further research into this subject has now shown that considerably better results can be obtained in the above-mentioned process if, as crystalline silicate with the previously mentioned special structure, a silicate containing gallium instead of aluminium is used, and if, moreover, the following two requirements are met:

a) in the formula giving the composition of the gallium silicate expressed in moles of the oxides, the $SiO_2/Ga_2O_3$ mol ratio should be 25—250, and

b) if the gallium silicate has a $SiO_2/Ga_2O_3$ mol ratio of 100—250, the catalyst should be subjected one or more times to a two-stage treatment comprising a first stage in which the catalyst is contacted for at least 15 minutes at a temperature of 350—700°C with a hydrogen-containing reducing gas, followed by a second stage in which the catalyst is contacted for at least 15 minutes at a temperature of 350—700°C with an oxygen-containing oxidizing gas.

The research showed that in the case of the present crystalline gallium silicates corresponding with the previously mentioned crystalline aluminium silicates, the higher the reaction temperature and/or the lower the $SiO_2/Ga_2O_3$ mol ratio of the silciate employed, the higher the aromatic content of the $C_5^+$ fracton of the product obtained, and also that for both measures the preparation of a product whose $C_5^+$ fraction has a higher aromatic content is accompanied by a reduction in the yield of the $C_5^+$ fraction. For the crystalline gallium silicates there is also a relationship between the increase in the aromatic content of the $C_5^+$ fraction and the reduction in the yield of the $C_5^+$ fraction, whereby, in principle, for every aromatic content of the $C_5^+$ fraction being prepared there is a particular potential yield of that $C_5^+$ fraction. The important difference between the crystalline aluminium silicates and the present crystalline gallium silicates is that the previously mentioned relationship for the gallium silicates is considerably more favourable, so that

2

considerably higher yields of $C_5^+$ fractions with a relatively high aromatic content can be obtained than is the case for the aluminium silicates. Comparison of the results obtained from a systematic investigation into processes for preparing aromatic hydrocarbons from a given feed, but employing a variety of aluminium silicates and gallium silicates at various temperatures showed that if the processes were aimed at the preparation of products whose $C_5^+$ fraction had a particular aromatic content, the gallium silicates gave a higher yield of the $C_5^+$ fraction, whereas if the processes were aimed at the preparation of products with a given content of the $C_5^+$ fraction, the gallium silicates gave a $C_5^+$ fraction with a higher aromatic content.

The present patent application therefore relates to a process for the preparation of an aromatic hydrocarbon mixture, wherein one or more mono-olefins with two to four carbon atoms per molecule or aliphatic hydrocarbons, at least 50%wt of which consisting of the said olefins, are contacted at a temperature of between 325 and 700°C with a catalyst containing a crystalline gallium silicate, which silicate has the following distinguishing features after calcination for one hour in air at 500°C:

a) an X-ray powder diffraction pattern that contains as strongest lines the four lines listed in Table A, and

b) in the formula which gives the composition of the silicate expressed in moles of the oxides, the $SiO_2/Ga_2O_3$ mol ratio is between 25 and 250,
and wherein, if the silicate has a $SiO_2/Ga_2O_3$ mol ratio of 100—250, the catalyst is subjected one or more times to the above-mentioned two-stage treatment.

The starting material for the process according to the invention should be one or more mono-olefins having two to four carbon atoms per molecule or aliphatic hydrocarbons, at least 50%wt of which consists of the said olefins. The mono-olefins with two to four carbon atoms per molecule, which should form at least 50%wt of the feed, are ethylene, propylene, butylene-1, butylene-2 and iso-butylene. If a hydrocarbon is employed that in addition to the said $C_4^-$ mono-olefins also contains other aliphatic hydrocarbons, these other hydrocarbons may be paraffins, di-olefins or $C_5^+$ mono-olfins. In the process according to the invention a feed is preferably employed which consists of at least 75%wt and in particular substantially completely of one or more mono-olefins with three or four carbon atoms per molecule. A very suitable feed for the present process is a hydrocarbon mixture consisting substantially of $C_3$ and/or $C_4$ mono-olefins, this mixture being obtained as byproduct of the catalytic or thermal cracking of hydrocarbons, in particular the steam cracking of hydrocarbons for the preparation of ethylene.

The process according to the invention is preferably carried out at a temperature of 350—575°C, a pressure of 1—20 bar and particularly 1—10 bar, and a space velocity of 0.1—10 $kg.kg^{-1}.hour^{-1}$ and in particular of 0.5—5 $kg.kg^{-1}.hour^{-1}$.

In the process according to the invention the feed is contacted with a catalyst containing a crystalline gallium silicate defined amongst other things by the X-ray powder diffraction pattern of the silicate after calcination for one hour in air at 500°C. This should contain as strongest lines the four lines given in Table A. The complete X-ray powder diffraction pattern of a typical example of the present crystalline gallium silicates after calcination for one hour in air at 500°C is represented in Table B.

3

EP 0 160 335 B1

TABLE B

| d(Å) | | Rel. int. | d(Å) | | Rel. int. |
|---|---|---|---|---|---|
| 11.1 | | 100 | 3.84 | (D) | 57 |
| 10.0 | (D) | 70 | 3.72 | (D) | 31 |
| 8.93 | | 1 | 3.63 | | 16 |
| 7.99 | | 1 | 3.47 | | < 1 |
| 7.42 | | 2 | 3.43 | | 5 |
| 6.68 | | 7 | 3.34 | | 2 |
| 6.35 | | 11 | 3.30 | | 5 |
| 5.97 | | 17 | 3.25 | | 1 |
| 5.70 | | 7 | 3.05 | | 8 |
| 5.56 | | 10 | 2.98 | | 11 |
| 5.35 | | 2 | 2.96 | | 3 |
| 4.98 | (D) | 6 | 2.86 | | 2 |
| 4.60 | | 4 | 2.73 | | 2 |
| 4.35 | | 5 | 2.60 | | 2 |
| 4.25 | | 7 | 2.48 | | 3 |
| 4.07 | | 2 | 2.40 | | 2 |
| 4.00 | | 4 | | | |

(D) = doublet

The catalysts for use in the process according to the invention are those containing a crystalline gallium silicate with a $SiO_2/Ga_2O_3$ mol ratio of 25—250. If the crystalline gallium silicate has a $SiO_2/Ga_2O_3$ mol ratio of 100—250, it should be subjected one or more times to the above-described two-stage treatment. Although crystalline gallium silicates with a $SiO_2/Ga_2O_3$ mol ratio of less than 100 are suitable as such for use in the process according to the invention, they are also preferably subjected one or more times to the above-described two-stage treatment. In the process according to the invention, preferred crystalline gallium silicates have a $SiO_2/Ga_2O_3$ mol ratio of 60—220. When employing a crystalline gallium silicate with a maximum $SiO_2/Ga_2O_3$ mol ratio of 110, the two-stage treatment is preferably not performed more than three times. When employing a crystalline gallium silicate with a $SiO_2/Ga_2O_3$ mol ratio of more than 110, but not more than 130, the two-stage treatment is preferably performed at least three times, but not more than ten times. When employing a crystalline gallium silicate with a $SiO_2/Ga_2O_3$ mol ratio of more than 130, but not more than 220, the silicate is preferably first subjected to calcination at a temperature of 600—1000°C, after which the two-stage treatment is performed on it at least three times, but not more than ten times.

During the first stage of the two-stage treatment the catalyst should be contacted for at least 15 times at a temperature of 350—700°C with a hydrogen-containing reducing gas. The first stage of the two-stage treatment can in principle be performed in two ways. In the first place the hydrogen-containing reducing gas can be fed to the catalyst from outside. In that case a gas is preferably employed that contains at least 20 vol.% and in particular at least 40 vol.% hydrogen. The gas employed can very suitably contain, besides hydrogen, either mainly nitrogen or mainly carbon monoxide or mainly $C_4^-$ hydrocarbons. Suitable gases which, besides hydrogen, contain mainly carbon monoxide can be obtained as synthesis gas from a highly carbonaceous material, such as coal, by gasification, or from light hydrocarbons, such as natural gas, by steam reforming or partial oxidation. Suitable gases which, besides hydrogen, contain mainly $C_4^-$ hydrocarbons can be obtained as byproduct from the catalytic conversion of hydrocarbons in the presence of hydrogen, such as cracking, isomerization and reforming. In the second place the hydrogen-containing reducing gas can be produced in situ by contacting the catalyst for a maximum of five hours with a hydrocarbon or a hydrocarbon mixture. A very suitable hydrocarbon or hydrocarbon mixture would in this case be the feed employed in the present process.

In the second stage of the two-stage treatment the catalyst should be contacted for at least 15 minutes at a temperature of 350—700°C with an oxygen-containing oxidizing gas. The gas employed preferably

4

contains at least 5 vol.% and in particular at least 10 vol.% oxygen. The second stage can very suitably be performed using a gas which, besides oxygen, contains either mainly nitrogen or otherwise mainly nitrogen, carbon monoxide and carbon dioxide. A suitable gas that, besides oxygen, contains mainly nitrogen is air. Suitable gases which, besides oxygen contain mainly nitrogen, carbon monoxide and carbon dioxide are offgases obtained during the removal of carbon by means of an excess of air from deactivated hydrocarbon conversion catalysts. Preferably, the two stages of the two-stage treatment are performed at a temperature of 400—650°C and in particular at a temperature of 475—575°C. It is also preferable that the two stages be performed at the same temperature.

The crystalline gallium silicates employed in the process according to the invention can very suitably be prepared from an aqueous mixture containing the following compounds: one or more compounds of an alkali metal (M), one or more organic nitrogen compounds (RN) containing an organic cation or from which an organic cation is formed during the preparation of the silicate, one or more silicon compounds and one or more gallium compounds. Preparation is effected by maintaining the mixture at an elevated temperature until the silicate has been formed and then separating the silicate crystals from the mother liquor and washing, drying and calcining the crystals. The aqueous mixture from which the silicates are prepared should contain the various compounds in the following molar ratios, except for the organic nitrogen compounds, expressed in moles of the oxides:

$M_2O:SiO_2 = 0.01—0.35$,
$RN:SiO_2 = 0.02—1.0$,
$SiO_2:Ga_2O_3 = 25—400$, and
$H_2O:SiO_2 = 5—65$.

The silicates can very suitably be prepared from a basis mixture containing a quaternary ammonium compound as organic nitrogen compound, a sodium compound as alkali metal compound and amorphous silica as silicon compound.

The silicates prepared according to the above method contain alkali metal ions. By means of suitable exchange techniques these can be replaced by other cations, such as hydrogen ions or ammonium ions. The crystalline gallium silicates employed in the process according to the invention preferably have an alkali metal content of less than 0.05%wt. In the process according to the invention the crystalline gallium silicates can be used as such or in combination with a binding material, such as kaolin or bentonite.

The invention will now be illustrated with the aid of the following example.

## Example

Two crystalline aluminium silicates (silicates 1 and 2) and three crystalline gallium silicates (silicates 3—5) were prepared by heating mixtures of NaOH, amorphous silica, $(C_3H_7)_4NOH$ and either $Al(NO_3)_3$, or $Ga(NO_3)_3$ in water in an autoclave under autogenous pressure for 24 hours at 150°C. After the reactants had cooled down, the resulting silicates were filtered off, washed with water until the pH of the wash water was about 8, and dried at 120°C. After calcination for one hour in air at 500°C, silicates 1—5 had the following properties:

a) an X-ray powder diffraction pattern corresponding substantially with that given in Table B, and
b) a value for the $SiO_2/Al_2O_3$ or $SiO_2/Ga_2O_3$ mol ratio as given in Table C.

### TABLE C

| Silicate No. | $SiO_2/Al_2O_3$ mol. ratio | $SiO_2/Ga_2O_3$ mol. ratio |
|---|---|---|
| 1 | 77 | - |
| 2 | 250 | - |
| 3 | - | 70 |
| 4 | - | 130 |
| 5 | - | 350 |

From silicates 1—5 were prepared silicates I—V by boiling silicates 1—5 with 1.0 molar $NH_4NO_3$ solution, washing with water, again boiling with 1.0 molar $NH_4NO_3$ solution, and washing, drying at 120°C and calcining at 500°C.

Samples of silicates III—V were repeatedly subjected to a two-stage treatment comprising a first stage in which the silicate was contacted for 30 minutes with n-butane at a temperature of 550°C, a pressure of 1.5 bar and a space velocity of 8 $g.g^{-1}.hour^{-1}$, followed by a second stage in which the silicate was contacted for one hour with air at a temperature of 550°C and a pressure of 1.5 bar. By performing this two-stage treatment three times on silicate III and ten .times on silicates IV and V, silicates IIIA—VA were obtained from silicates III—V respectively.

Silicates I, II, IIIA, IV, IVA and VA were tested in twelve experiments (experiments 1—12) for the preparation of $C_5^+$ fractions from iso-butylene (experiments 1—11) and from propylene (experiment 12). All experiments were performed at a pressure of 1.5 bar and a space velocity of 2 $kg.kg^{-1}.hour^{-1}$ in a reactor containing a solid catalyst. Conversion of the feed was practically complete in all the experiments. The temperatures at which the experiments were carried out and the results of these experiments are given in Table D.

Of experiments 1—12, only experiments 5—7, 9 and 12 are experiments according to the invention. The other experiments fall outside the scope of the invention. They are included in the patent application for the purpose of comparison. No crystalline gallium silicate was used in the comparative experiments 1—4. In the comparative experiments 8, 10 and 11, despite the fact that a crystalline gallium silicate was used, the results were nevertheless unsatisfactory, the reasons being that the silicate was not pre-treated in experiment 8, the reaction temperature was too low in experiment 10, and the gallium silicate had a too high $SiO_2/Ga_2O_3$ mol ratio in experiment 11.

A comparison of experiment 7 (with gallium silicate) and experiment 3 (with aluminium silicate) shows that for an equal yield of $C_5^+$ fraction (63%wt) the gallium silicate produces a $C_5^+$ fraction with a much higher aromatic content (83%wt) than the aluminium silicate (48%wt).

A comparison of experiment 7 (with gallium silicate and experiment 4 (with aluminium silicate shows that for an almost identical aromatic content of the $C_5^+$ fraction (81—83%wt) the gallium silicate gives a much higher yield of $C_5^+$ fraction (63%wt) than the aluminium silicate (38%wt). This was also shown by a comparison of experiments 5 and 3.

## TABLE D

| Experiment No. | 1 | 2 | 3 | 4 | 5 | 6 | 7 | 8 | 9 | 10 | 11 | 12 |
|---|---|---|---|---|---|---|---|---|---|---|---|---|
| Silicate No. | II | I | II | II | IIIA | IIIA | IIIA | IV | IVA | IIIA | VA | IIIA |
| Temperature, °C | 400 | 400 | 450 | 575 | 350 | 425 | 550 | 425 | 425 | 315 | 500 | 550 |
| Yield of $C_4^-$ fraction, %wt | 16 | 37 | 37 | 62 | 28 | 33 | 37 | 36 | 30 | 3 | 67 | 39 |
| Yield of $C_5^+$ fraction, %wt | 84 | 63 | 63 | 38 | 72 | 67 | 63 | 64 | 70 | 97 | 33 | 61 |
| Aromatic content of $C_5^+$ fraction, %wt | 19 | 49 | 48 | 81 | 48 | 65 | 83 | 48 | 52 | 2 | 11 | 85 |

EP 0 160 335 B1

## Claims

1. Process for the preparation of an aromatic hydrocarbon mixture, characterized in that one or more olefins with two to four carbon atoms per molecule or aliphatic hydrocarbons, at least 50%wt of which consisting of the said olefins, are contacted at a temperature of between 325 and 700°C with a catalyst containing a crystalline gallium silicate, which silicate has the following distinguishing features after calcination for one hour in air at 500°C:

a) an X-ray powder diffraction pattern that contains as strongest lines the four lines listed in Table A, and

b) in the formula which gives the composition of the silicate expressed in moles of the oxides, the $SiO_2/Ga_2O_3$ mol ratio is between 25 and 250,

and that, if the silicate has a $SiO_2/Ga_2O_3$ mol ratio of 100—250, the catalyst is subjected one or more times to a two-stage treatment comprising a first stage in which the catalyst is contacted for at least 15 minutes at a temperature of 350—700°C with a hydrogen-containing reducing gas, followed by a second stage in which the catalyst is contacted for at least 15 minutes at a temperature of 350—700°C with an oxygen-containing oxidizing gas.

2. Process according to claim 1, characterized in that it is applied to a feed, 75%wt of which consists of one or more mono-olefins with three or four carbon atoms per molecule.

3. Process according to claim 2, characterized in that the feed consists substantially completely of one or more mono-olefins with three or four carbon atoms per molecule.

4. Process according to claim 3, characterized in that the feed is a hydrocarbon mixture consisting substantially of $C_3$ and/or $C_4$ mono-olefins, this mixture being obtained as byproduct of the catalytic or thermal cracking of hydrocarbons.

5. Process according to one of claims 1—4, characterized in that it is carried out at a pressure of 1—20 bar and a space velocity of 0.1—10 $kg.kg^{-1}.hour^{-1}$.

6. Process according to claims 1—5, characterized in that a crystalline gallium silicate is used with a $SiO_2/Ga_2O_3$ mol ratio of 60—220.

7. Process according to one or more of claims 1—6, characterized in that the crystaline gallium silicate is prepared from an aqueous mixture containing the following compounds: one or more compounds of an alkali metal (M), one or more organic nitrogen compounds (RN) containing an organic cation or from which an organic cation is formed during the preparation of the silicate, one or more silicon compounds and one or more gallium compounds, and in which mixture the various compounds are present in the following molar ratios, except for the organic nitrogen compounds, expressed in moles of the oxides:

$M_2O:SiO_2 = 0.01—0.35$,
$RN:SiO_2 = 0.02—1.0$,
$SiO_2:Ga_2O_3 = 25—400$, and
$H_2O:SiO_2 = 5—65$

by maintaining the aqueous mixture at an elevated temperature until the silicate has been formed and then separating the silicate crystals from the mother liquor and calcining them.

8. Process according to one of claims 1—7, characterized in that the crystalline gallium silicate has an alkali metal content of less than 0.05%wt.

## Patentansprüche

1. Verfahren zur Herstellung einer aromatischen Kohlenwasserstoffmischung, dadurch gekennzeichnet, daß ein oder mehrere Olefin(e) mit zwei bis vier Kohlenstoffatomen pro Molekül oder aliphatische Kohlenwasserstoffe, welche zumindest zu 50 Gewichtsprozent aus diesen Olefinen bestehen, bei einer Temperatur von 325 bis 700°C mit einem ein kristallines Galliumsilikat enthaltenden Katalysator kontaktiert wird (werden) welches Silikat nach einer einstündigen Kalzinierung an Luft bei 500°C die folgenden Unterscheidungsmerkmale aufweist:

a) ein Röntgen-Pulverbeugungsdiagramm, welches als stärkste Linien die vier in Tabelle A genannten Linien aufweist, und

b) in der Formel für die Zusammensetzung des Silikats, ausgedrückt in Mol der Oxide, beträgt das Molverhältnis von $SiO_2/Ga_2O_3$ zwischen 25:1 und 250:1,

und daß, wenn das Silikat ein Molverhältnis von $SiO_2/Ga_2O_3$ von 100:1 bis 250:1 aufweist, der Katalysator einmal oder mehrmals einer Zweistufenbehandlung unterworfen wird, wobei in der ersten Stufe der Katalysator mindestens 15 Minuten lang bei einer Temperatur von 350 bis 700°C mit einem Wasserstoff enthaltenden reduzierenden Gas kontaktiert wird und in der zweiten Stufe der Katalysator mindestens 15 Minuten lang bei einer Temperatur von 350 bis 700°C mit einem Sauerstoff enthaltenden oxidierenden Gas kontaktiert wird.

2. Verfahren nach Anspruch 1, dadurch gekennzeichnet, daß es für eine Zuspeisung angewendet wird, die zu 75 Gewichtsprozent aus einem oder mehreren Monoolefin(en) mit 3 oder 4 Kohlenstoffatomen pro Molekül besteht.

3. Verfahren nach Anspruch 2, dadurch gekennzeichnet, daß die Zuspeisung im wesentlichen vollständig aus einem oder mehreren Monoolefin(en) mit 3 oder 4 Kohlenstoffatomen pro Molekül besteht.

4. Verfahren nach Anspruch 3, dadurch gekennzeichnet, daß die Zuspeisung eine Kohlenwasserstoffmischung bestehend aus im wesentlichen $C_3$- und/oder $C_4$-Monoolefinen ist, wobei diese Mischung als Nebenprodukt beim katalytischen oder thermischen Cracken von Kohlenwasserstoffen erhalten wird.

5. Verfahren nach einem der Ansprüche 1 bis 4, dadurch gekennzeichnet, daß es bei einem Druck von 1 bis 20 bar und bei einer Raumgeschwindigkeit von 0,1 bis 10 kg/kg$^{-1}$. Stunde$^{-1}$ durchgeführt wird.

6. Verfahren nach einem der Ansprüche 1 bis 5, dadurch gekennzeichnet, daß ein kristallines Galliumsilikat mit einem Mol verhältnis von $SiO_2/Ga_2O_3$ von 60:1 bis 220:1 verwendet wird.

7. Verfahren nach einem oder mehreren der Ansprüche 1 bis 6, dadurch gekennzeichnet, daß das kristalline Galliumsilikat aus einer wäßrigen Mischung, enthaltend die folgenden Verbindungen, hergestellt wurde: eine oder mehrere Verbindung(en) eines Alkalimetalls(M), eine oder mehrere organische Stickstoffverbindung(en) (RN) enthaltend ein organisches Kation, oder aus welcher(n) während der Herstellung des Silikats ein organisches Kation gebildet wird, eine oder mehrere Siliciumverbindung(en) und eine oder mehrere Galliumverbindung(en), und in welcher Mischung die verschiedenen Verbindungen in den folgenden molaren Verhältnissen vorliegen, mit Ausnahme der organischen Stickstoffverbindung(en) ausgedrückt in Mol der Oxide:

$M_2O:SiO_2$ = 0,01:1—0,35:1,
$RN:SiO_2$ = 0,02:1—1,0:1,
$SiO_2:Ga_2O_3$ = 25:1—400:1, und
$H_2O:SiO_2$ = 5:1—65:1,

wobei die wäßrige Mischung auf erhöhter Temperatur gehalten wird, bis das Silikat gebildet ist, und dann die Silikatkristalle aus der Mutterlauge abgetrennt und calziniert werden.

8. Verfahren nach einem der Ansprüche 1 bis 7, dadurch gekennzeichnet, daß das kristalline Galliumsilikat einen Alkalimetallgehalt von weniger als 0,05 Gewichtsprozent aufweist.

**Revendications**

1. Procédé pour la préparation d'un mélange d'hydrocarbures aromatiques, caractérisé en ce que ou plusieurs mono-oléfines ayant de 2 à 4 atomes de carbone par molécule, ou un ou plusieurs hydrocarbures aliphatiques, dont au moins 50% en poids est constitué par lesdites oléfines, sont mis en contact à une température comprise entre 325 et 700°C avec un catalyseur contenant un silicate de gallium cristallin, lequel silicate a les caractéristiques distinctives sivantes, après calcination pendant une heure à l'air à 500°C:

a) un spectre de diffraction des rayons X de la poudre qui contient comme raies principales les quatre raies énumérées dans le tableau A et,

b) dans la formule qui donne la composition du silicate exprimée en moles d'oxydes, le rapport molaire $SiO_2/Ga_2O_3$ est compris entre 25 et 250, et en ce que, si le silicate a un rapport molaire $SiO_2/Ga_2O_3$ de 100 à 250, le catalyseur est soumis une ou plusieurs fois à un traitement en deux étapes comprenant une première étape au cours de laquelle le catalyseur est mis en contact, pendant au moins 15 minutes, à une température de 350 à 700°C, avec un gaz réducteur contenant de l'hydrogène, suivie par une seconde étape au cours de laquelle le catalyseur est mis en contact pendant au moins 15 minutes, à une température de 350 à 700°C, avec un gaz oxydant contenant de l'oxygène.

2. Procédé selon la revendication 1, caractérisé en ce qu'il est appliqué à une alimentation dont 75% en poids est constitué à une alimentation dont 75% en poids est constitué par une ou plusieurs mono-oléfines ayant 3 ou 4 atomes de carbone par molécule.

3. Procédé selon la revendication 2, caractérisé en ce que l'alimentation est constituée presque totalement d'une ou plusieurs mono-oléfines ayant 3 ou 4 atomes de carbone par molécule.

4. Procédé selon la revendication 3, caractérisé en ce que l'alimentation est un mélange d'hydrocarbures consistant essentiellement en mono-oléfines en $C_3$ et/ou en $C_4$, ce mélange étant obtenu comme sous-produit du craquage thermique ou catalytique d'hydrocarbures.

5. Procédé selon l'une des revendications 1 à 4, caractérisé en ce qu'il est mis en oeuvre à une pression de 1 à 20 bars et à une vitesse spatiale de 0,1 à 10 kg.kg$^{-1}$.heure$^{-1}$.

6. Procédé selon l'une des revendications 1 à 5, caractérisé en ce qu'on utilise un silicate de gallium cristallin avec un rapport molaire $SiO_2/Ga_2O_3$ de 60 à 220.

7. Procédé selon une ou plusieurs des revendications 1 à 6, caractérisé en ce que le silicate de gallium cristallin est préparé à partir d'un mélange aqueux contenant les composés suivants: un ou plusieurs composés d'un métal alcalin (M), un ou plusieurs composés azotés organiques (RN) contenant un cation organique ou à partir duquel un cation organique est formé pendant la préparation du silicate, un ou plusieurs composés du silicium et un ou plusieurs composés du gallium, et dans lequel les différents composés sont présents dans les rapports molaires suivants, excepté pour les composés azotés organiques, exprimés en moles d'oxydes:

$M_2O:SiO_2 = 0{,}01{-}0{,}35,$
$RN:SiO_2 = 0{,}02{-}1{,}0,$
$SiO_2:Ga_2O_3 = 25{-}400,$ et
$H_2O:SiO_2 = 5{-}65,$

par maintien du mélange aqueux à une température élevée jusqu'à ce que le silicate soit formé et ensuite par séparation des cristaux de silicate d'avec la liqueur mère et par calcination de ces cristaux.

8. Procédé selon l'une des revendications 1 à 7, caractérisé en ce que le silicate de gallium cristallin a une teneur en métal alcalin inférieure à 0,05% en poids.